# EUROPÄISCHE PATENTSCHRIFT

(11) **EP 3 302 612 B1**
(45) Veröffentlichungstag und Bekanntmachung des Hinweises auf die Patenterteilung: **14.08.2019**
(21) Anmeldenummer: 16726847.3
(22) Anmeldetag: 27.05.2016
(51) Int. Cl.: A61M 1/16, A61M 39/10

(54) **DIALYSEKONZENTRAT-HERSTELLUNGSANORDNUNG**
DIALYSIS-CONCENTRATE PRODUCTION ASSEMBLY
DISPOSITIF DE PRODUCTION D'UN CONCENTRÉ DE DIALYSE

(30) Priorität: 27.05.2015 DE 202015102734 U
(43) Veröffentlichungstag der Anmeldung: 11.04.2018
(73) Patentinhaber: INTERMEDT Medizin & Technik GmbH, 26842 Ostrhauderfehn (DE)
(72) Erfinder: DUMSCHAT, Christoph, 126789 Leer (DE)
(74) Vertreter: Patentanwälte ter Smitten Eberlein-Van Hoof Rütten Partnerschaftsgesellschaft mbB
(86) Internationale Anmeldenummer: PCT/EP2016/062083
(87) Internationale Veröffentlichungsnummer: WO 2016/189161

(56) Entgegenhaltungen:
- DE-B3- 10 313 965
- US-A1- 2003 168 120
- US-A1- 2014 191 501
- US-A1- 2015 083 647

## Beschreibung

Die Erfindung bezieht sich auf eine Dialysekonzentrat-Herstellungsanordnung zur Herstellung einer Dialysekonzentrat-Flüssigkeit durch Lösen eines Trockenkonzentrats in Wasser.

Für die Durchführung einer typischen Dialysebehandlung werden üblicherweise 150 bis 200I Dialyseflüssigkeit benötigt. Die Dialyseflüssigkeit wird in der Regel in dem Dialysegerät aus zwei Dialysekonzentratflüssigkeiten und Wasser erzeugt, die beispielsweise in einem Verhältnis 1/35 miteinander vemischt werden. Für die Herstellung der Dialyseflüssigkeit wiederum werden eine basische Dialysekonzentratflüssigkeit, die üblicherweise aus einer Natriumhydrogenkarbonat-Lösung definierter Konzentration besteht, und eine saure Dialysekonzentratflüssigkeit, die alle übrigen für die Dialyseflüssigkeit erforderlichen Bestandteile in der erforderlichen Konzentration enthält, mit Wasser homogen vermischt.

Aus DE 103 13 965 B3 ist eine Dialysekonzentrat-Herstellungsanordnung bekannt, durch die zur Herstellung der Dialysekonzentratflüssigkeit ein Trockenkonzentrat mit Wasser homogen vermischt wird. Das Trockenkonzentrat wird in einem mobilen und mehrfach verwendbaren Wechselbehälter geliefert, der zwei Flüssigkeits-Anschlüsse aufweist, nämlich einen Kombianschluss, der in beiden Richtungen benutzt werden kann, und einen Ablaufanschluss, durch den Flüssigkeit aus dem Wechselbehälter ablaufen kann. Die beiden Anschlüsse werden am Einsatzort an eine stationäre Herstellungsanlage angeschlossen. Anschließend wird durch entsprechendes Pumpen und Leiten von Wasser das Trockenkonzentrat in Wasser gelöst und homogen zu der Dialysekonzentrat-Flüssigkeit vermischt. Die Flüssigkeit wird hierbei unter einem gewissen Druck in den Wechselbehälter hineingepumpt und fließt unter Druck aus dem Ablaufanschluss heraus.

Das fluidische Anschließen der Herstellungsanlage an die beiden Anschlüsse erfolgt jeweils durch eine Kupplungsanordnung, die keine Besonderheiten aufweist, so dass grundsätzlich die Gefahr von Fehlbedienungen und Leckagen besteht.

Aus US 2003/0168120 A1, US 2015/0083647 A1 und DE 103 13 965 B3 sind Dialysekonzentrat-Herstellungsanordnungen mit einem mobilen Wechselbehälter und einer stationären Herstellungsanlage bekannt. Zur fluidischen Verbindung des Wechselbehälters mit der Herstellungsanlage sind fluidische Kupplungsanordnungen vorgesehen, die sich ausschließlich auf Flüssigkeitsleitungen beziehen. Eine Drucküberwachung des Fluiddruckes innerhalb des Wechselbehälters ist nicht vorgesehen.

Aufgabe der Erfindung ist es demgegenüber, eine Dialysekonzentrat-Herstellungsanordnung mit bedienungssicheren Kupplungsanordnungen mit guten Dichtungseigenschaften zu schaffen. Aufgabe der Erfindung ist es ferner, einen Wechselbehälter für eine Dialysekonzentrat-Herstellungsanordnung zu schaffen, der sich bedienungssicher und mit hoher fluidischer Dichtigkeit an die Herstellungsanlage anschließen lässt.

Diese Aufgabe wird erfindungsgemäß gelöst mit einer Dialysekonzentrat-Herstellungsanordnung mit den Merkmalen des Anspruchs 1 bzw. mit einem Wechselbehälter für eine Dialysekonzentrat-Herstellungsanordnung, mit den sich auf den Wechselbehälter beziehenden Merkmalen des Anspruchs 1.

Der erfindungsgemäße Wechselbehälter, in dem das Trockenkonzentrat gelagert ist, weist einen Ablaufanschluss und einen Kombianschluss auf.

Die beiden Anschlüsse sind über jeweils eine Kupplungsanordnung mit der stationären Herstellungsanlage fluidisch verbunden. Jede Kupplungsanordnung weist jeweils einen anlageseitigen Kupplungskörper und einen behälterseitigen Kupplungskörper auf. Die Kupplungskörper sind komplexe Strukturen, die sowohl die mechanische als auch die fluidische Verbindung herstellen. Der behälterseitige Kupplungskörper weist eine im Querschnitt nicht-kreisförmige Flüssigkeits-Leitungsbuchse auf, die mit einem entsprechenden im Querschnitt nicht-kreisförmigen und komplementären Flüssigkeits-Leistungsstecker des anlageseitigen Kupplungskörpers korrespondiert und zusammengesteckt ist. Der Flüssigkeits-Leitungsstecker und die Flüssigkeits-Leitungsbuchse sind derart geformt, dass sie nur in einer einzigen rotatorischen Position ineinander steckbar sind. Alternativ kann der Flüssigkeits-Leitungsstecker auch an dem behälterseitigen Kupplungskörper und die Flüssigkeits-Leitungsbuchse an dem anlageseitigen Kupplungskörper vorgesehen sein. Besonders bevorzugt sind die Leitungsbuchse und der Leitungsstecker der Ablaufanschluss-Kupplungsanordnung verschieden von der Leitungsbuchse und dem Leitungsstecker der Kombianschluss-Kupplungsanordnung ausgebildet. Auf diese Weise wird nicht nur ein betriebssicheres und bedienungssicheres Zusammenstecken der Kupplungsanordnung sichergestellt, sondern auch ein Vertauschen der Anschlüsse für den Ablaufanschluss und den Kombianschluss ausgeschlossen.

Durch Festlegen einer einzigen rotatorischen Position für das Zusammenstecken der Leitungsbuchse mit dem Leitungsstecker kann ein Verdrehen der Leitungsbuchse und des Leitungssteckers zueinander vor und während des Zusammensteckens, während des Betriebes bzw. während des Lösens der jeweiligen Kupplungsanordnung ausgeschlossen werden. Hierdurch werden insbesondere Dichtungen, Dichtringe etc. erheblich geschont, wodurch die fluidische Dichtigkeit auch bei mehrfacher Verwendung des Wechselbehälters sichergestellt ist. Gemäß einer bevorzugten Ausgestaltung sind der Flüssigkeits-Leitungsstecker und die Flüssigkeits-Leitungsbuchse jeweils oval ausgebildet. Hierdurch werden insbesondere enge Radien bezogen auf die Querschnittsform des Leitungssteckers und der Leitungsbuchse vermieden, so dass auch unter Druck eine zuverlässige fluiddichte Abdichtung der Kupplungsanordnung sichergestellt werden kann, gleichzeitig jedoch eine nicht-kreisförmige Querschnittsform realisiert wird, die ein Ineinanderstecken der Leitungsbuchse und des Leitungssteckers nur in einer einzigen rotatorischen Position zulässt.

Die Herstellungsanlage weist einen fluidischen Drucksensor auf, der fluidisch über eine Leitung mit einer der beiden Kupplungsanordnungen verbunden ist, die anlageseitig einen Drucksensor-Leitungsstecker und behälterseitig eine korrespondierende Drucksensor-Leitungsbuchse aufweist. Selbstverständlich kann die Leitungsbuchse alternativ auch anlagenseitig und kann der Leitungsstecker behälterseitig vorgesehen sein. Der jeweilige Kupplungskörper weist also zwei Leitungsstecker bzw. Leitungsbuchsen auf, nämlich einen Leitungsstecker für die Flüssigkeit und einen pneumatischen Leitungsstecker. Über die pneumatische Verbindung von dem Wechselbehälter-Innenraum zu dem Drucksensor der Herstellungsanlage kann während des Mischvorgangs der Innendruck in dem Wechselbehälter ständig überwacht werden, so dass der Innendruck in dem Wechselbehälter durch Steuern entsprechender Flüssigkeits-Pumpen in der Herstellungsanlage optimal eingestellt und geregelt werden kann. Hierdurch kann zum Einen eine gewisse Mischintensität sichergestellt werden und kann zum Anderen insbesondere ein relativ niedriger Überdruck in dem Wechselbehälter eingestellt werden, so dass überdruckbedingte Leckagen bzw. Beschädigungen vermieden werden können.

Vorzugsweise weist der Flüssigkeits-Leitungsstecker außenseitig eine in axialer Richtung langgestreckte Ringnut auf, in der ein flexibler O-Ring sitzt. Die Ringnut weist eine axiale Länge auf, die mindestens das 1,5-fache des Durchmessers des Körpers des O-Rings beträgt, besonders bevorzugt mindestens das 2,0-fache. Der O-Ring liegt also mit seiner radialen Innenseite an dem Boden der Ringnut an. Beim Einstecken des Flüssigkeits-Leitungssteckers in die korrespondierende Flüssigkeits-Leitungsbuchse kommt der O-Ring mit seiner radialen Außenseite in Kontakt mit einem zylindrischen Dichtabschnitt der Leitungsbuchse, so dass beim weiteren Zusammenstecken der O-Ring zwischen den beiden zylindrischen Flächen der Ringnut und der Leitungsbuchse abgerollt wird, und zwar ungefähr mit der halben Geschwindigkeit der axialen Steckbewegung. Hierdurch wird sowohl beim Zusammenstecken als auch beim Lösen der betreffenden Kupplungsanordnung die Reibung an dem O-Ring minimiert, so dass zum Einen der mechanische Verschleiß an dem O-Ring minimiert wird und zum Anderen wegen der geringen Reibung das Ein- und Ausstecken nur geringe Steckkräfte erfordert.

Gemäß einer bevorzugten Ausgestaltung bestehen die Kupplungskörper der Kupplungsanordnung aus Kunststoff, besonders bevorzugt aus einem Kunststoff ohne Faserverstärkung. Die Kupplungskörper bestehen also nicht aus Metall, das in Bezug auf die in der Dialysekonzentrat-Flüssigkeit enthaltenen Chloride und Säuren korrosiv sein kann bzw. aufwändig dagegen geschützt werden müsste.

Gemäß einer bevorzugten Ausgestaltung weist der behälterseitige Kupplungskörper einen RFID-Identifikationssender und der anlagenseitige Kupplungskörper einen RFID-Empfänger zum Lesen einer Identifikation aus dem RFID-Identifikationssender auf. Die in dem Identifikationssender gespeicherte Identifikation identifiziert bevorzugt den Wechselbehälter und/oder das in dem Wechselbehälter gespeicherte Trockenkonzentrat.

Beim Zusammenstecken der betreffenden Kupplungsanordnung kann durch die stationäre Herstellungsanlage über den RFID-Empfänger die Identifikation aus dem Identifikationssender abgefragt werden. Auf diese Weise kann beispielsweise sichergestellt werden, dass das Verfallsdatum des betreffenden Trockenkonzentrats noch nicht abgelaufen ist. Ferner können in der Identifikation auch inhaltliche Informationen über das Trockenkonzentrat gespeichert werden. Schließlich können über die Identifikation auch Angaben über die Herkunft, das Alter und die Häufigkeit der Wiederverwendungen des Wechselbehälters gespeichert werden.

Vorzugsweise kann der Flüssigkeits-Leitungsstecker und/oder die korrespondierende Flüssigkeits-Leitungsbuchse im Öffnungsbereich mit einer zirkulären Fase ausgestattet sein, durch die das Einfädeln bzw. das Ineinanderstecken der Leitungsbuchse mit dem Leitungsstecker erleichtert wird.

Vorzugsweise weist die Kupplungsanordnung eine Bajonett-Anordnung auf, durch die ein Bajonett-Verschluss realisiert wird. Insbesondere weist die Kupplungsanordnung an einem der beiden Kupplungskörper eine Überwurfmutter auf, die eine Seite der Bajonett-Anordnung aufweist. Die Bajonett-Anordnung weist einen kleinen Verriegelungswinkel von unter 70° auf, so dass die rotatorische Schließbewegung der Überwurfmutter höchstens 70° beträgt. Die Bajonett-Anordnung weist ferner eine hinterschnittene axiale Steigung auf, so dass die Überwurfmutter während der Verriegelungsbewegung axial verspannt wird. Ferner weist die Überwurfmutter mehrere radiale Rastnasen mit radialen Steigungen auf, durch die eine Zentrierung der Überwurfmutter und des damit verbundenen Kupplungskörpers realisiert wird.

Besonders bevorzugt weist die Überwurfmutter rotatorische Endanschläge auf, durch die die rotatorische Schließbewegung begrenzt ist.

Gemäß einer besonders bevorzugten Ausgestaltung weist der Wechselbehälter ein an den Kombianschluss anschließendes Steigrohr auf, das nach unten mindestens bis in das untere Drittel des Wechselbehälters ragt. Das Steigrohr ist in gleicher Weise oval ausgebildet, wie die Leitungsbuchse, durch die das Steigrohr mit dem betreffenden Leitungsstecker verbunden ist bzw. wird. Der Steigrohr- Körper bildet auch die Leitungsbuchse.

Im Folgenden wird unter Bezugnahme auf die Zeichnungen ein Ausführungsbeispiel der Erfindung näher erläutert. Es zeigen:
Figur 1 eine schematische Darstellung einer Herstellungsanordnung zur Herstellung einer Dialysekonzentrat-Flüssigkeit, mit einer stationären Herstellungsanlage und einem Wechselbehälter,
Figur 2 die Kupplungskörper der Kupplungsanordnungen zur fluidischen Verbindung des Wechselbehälters mit der Herstellungsanlage der Figur 1,
Figur 3 eine Draufsicht auf den Behälterdeckel des Wechselbehälters der Figur 1, und
Figur 4 einen Längsschnitt der Kupplungskörper der Ablaufanschluss-Kupplungsanordnung der Herstellungsanordnung der Figur 1.

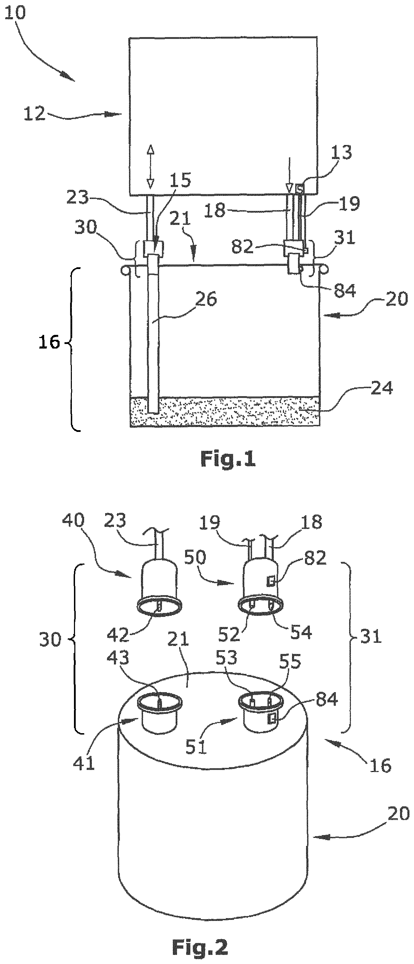

Die Figur 1 zeigt schematisch eine Dialysekonzentrat-Herstellungsanordnung 10 zur Herstellung einer Dialysekonzentrat-Flüssigkeit durch Lösen eines Trockenkonzentrats 24 in Wasser. Die Herstellungsanordnung 10 besteht aus einem mobilen Wechselbehälter 16 mit dem Trockenkonzentrat 24 und einer stationären Herstellungsanlage 12, die eine Vielzahl von Komponenten, insbesondere Ventile, Pumpen und eine Anlagensteuerung aufweist.

Der mobile Wechselbehälter 16 weist einen tonnenartigen Behälterkörper 20 auf, dessen Oberseite durch einen Behälterdeckel 21 fluiddicht verschlossen ist. An dem Behälterdeckel 21 weist der Wechselbehälter 16 zwei Flüssigkeitsanschlüsse auf, nämlich einen Kombianschluss 15 und einen Ablaufanschluss 17. Der Kombianschluss 15 des Wechselbehälters 16 ist über eine Kupplungsanordnung 30 und eine Flüssigkeitsleitung 23 fluidisch mit der Herstellungsanlage 12 verbunden. Der Ablaufanschluss 17 ist über eine Kupplungsanordnung 31 und eine Flüssigkeitsleitung 18 ebenfalls fluidisch mit der Herstellungsanlage 12 verbunden. Ferner ist die Ablaufanschluss-Kupplungsanordnung über eine pneumatische Druckleitung 19 mit einem Drucksensor 13 der Herstellungsanlage 12 pneumatisch verbunden. Ein RFID-Empfänger 82 der Ablaufanschluss-Kupplungsanordnung ist über eine Datenleitung mit der Herstellungsanlage 12 elektronisch verbunden.

Der Wechselbehälter 16 weist ein im Querschnitt ovales Steigrohr 26 auf, das mit dem Kombianschluss 15 verbunden ist bzw. in diesen mündet. Der Steigrohr-Körper bildet das Steigrohr 26 und eine behälterseitige Leitungsbuchse.

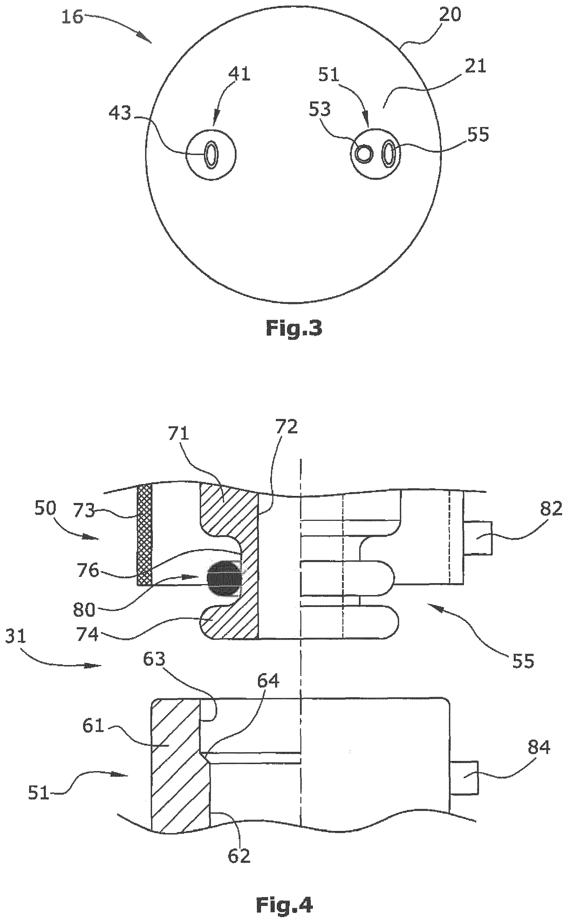

In der Figur 3 sind die beiden Kupplungsanordnungen 30, 31 nichtineinandergesteckt dargestellt. Links ist die Kombianschluss-Kupplungsanordnung 30 dargestellt, die von zwei Kupplungskörpern 40, 41 gebildet wird, nämlich von einem anlagenseitigen Kupplungskörper 40 mit einem im Querschnitt ovalen Leitungsstecker 42 und einem behälterseitigen Kupplungskörper 41 mit einer ebenfalls im Querschnitt ovalen Leitungsbuchse 43. Die Kupplungskörper 40, 41, der Leitungsstecker 42 und die Leitungsbuchse 43 sind derart gestaltet und angeordnet, dass die beiden Kupplungskörper 40, 41 nur in einer einzigen rotatorischen Position axial, also in Steckrichtung, ineinander steckbar sind. Dies kann beispielsweise dadurch realisiert sein, dass der ovale Leitungsstecker bzw. die korrespondierende ovale Leitungsbuchse nicht zentrisch in dem zylindrischen Gehäuse des jeweiligen Kupplungskörpers 40, 41 angeordnet sind.

Die Ablaufanschluss-Kupplungsanordnung 31 weist einen anlagenseitigen Kupplungskörper 50 und einen behälterseitigen Kupplungskörper 51 auf. Der behälterseitige Kupplungskörper 51 weist in dem zylindrischen Kupplungsgehäuse eine Flüssigkeits-Leitungsbuchse auf, die mit einem entsprechenden Flüssigkeits-Leitungsstecker 54 des anlagenseitigen Kupplungskörpers 50 korrespondiert. Der Leitungsstecker 54 und die Leitungsbuchse 55 sind im Querschnitt oval ausgebildet und exzentrisch in dem jeweiligen Kupplungskörper angeordnet.

Die Ablaufanschluss-Kupplungsanordnung 31 weist ferner an dem anlageseitigen Kupplungskörper 50 einen Drucksensor-Leitungsstecker 52 und an dem behälterseitigen Kupplungskörper 51 eine korrespondierende Drucksensor-Leitungsbuchse 53 auf. Hierdurch wird eine pneumatische Verbindung von dem Wechselbehälter-Innenraum zu dem anlagenseitigen Drucksensor 13 hergestellt.

An der Außenseite des Ablaufanschluss-Kupplungskörpers 51 ist ein RFID-Identifikationssender 84 fixiert. An dem anlagenseitigen Kupplungskörper 50 ist ein RFID-Empfänger 82 angeordnet, der die Identifikation bzw. Identifikationsdaten aus dem Identifikationssender 84 auslesen kann, wenn die Kupplungskörper 51 zusammengesteckt sind. In dem Identifikationssender 84 sind insbesondere eine eindeutige Identifikation des Wechselbehälters 16, Informationen über die Haltbarkeit und Zusammensetzung des Trockenkonzentrats 24 sowie Haltbarkeitsinformationen über den Wechselbehälter 16 gespeichert.

In der Figur 4 sind die beiden Kupplungskörper 50, 51 der Ablaufanschluss-Kupplungsanordnung 31 teilweise im Längsschnitt dargestellt. Der Kupplungskörper 50 weist ein zylindrisches Kupplungsgehäuse 73 auf, in dem nicht-zentrisch die Flüssigkeits-Leitungsbuchse 55 angeordnet ist, die von einem im Wesentlichen zylindrischen Seckerkörper 71 gebildet wird, der eine zylindrische Innenseite 72 und eine im Wesentlichen zylindrische Außenseite aufweist. Der Steckerkörper 71 weist an seiner Außenseite endseitig eine in Steckrichtung bzw. in axialer Richtung langgestreckte Ringnut 76 auf, in der ein O-Ring 80 abrollbar sitzt. Die Ringnut 76 wird endseitig begrenzt durch einen entsprechenden Ringwulst 74 des Steckerkörpers 71. Die axiale Länge der Ringnut 76 beträgt ungefähr das Dreifache des Durchmessers des O-Rings 80, so dass dieser etwa einmal vollständig darin abrollen kann.

Der korrespondierende Kupplungskörper 51 des Wechselbehälters 16 weist einen im Wesentlichen zylindrischen Steckerkörper 61 auf, dessen Außendurchmesser geringfügig kleiner ist als der Innendurchmesser des anlagenseitigen Steckergehäuses 73. An der offenen Seite weist der Buchsenkörper 61 eine Steckbohrung 63 auf, in die der Steckerkörper 71 eingesteckt werden kann, wobei der O-Ring geringfügig komprimiert und gleichzeitig zwischen der Bodenwand der Ringnut 76 und der zylindrischen Innenwand 63 des Buchsenkörpers 61 abgerollt wird. Der Buchsenkörper 61 weist innenseitig eine Stufe 64 auf, durch die sich der Innenumfang auf eine Bohrung 62 mit geringerem Innendurchmesser verjüngt.

Die Kupplungskörper 40, 50, 41, 51 der Kupplungsanordnungen 30, 31 bestehen komplett aus Kunststoff.

## Patentansprüche

1. Dialysekonzentrat-Herstellungsanordnung (10) zur Herstellung einer Dialysekonzentrat-Flüssigkeit durch Lösen eines Trockenkonzentrats (24) in Wasser, mit
einem mobilen Wechselbehälter (16) mit dem Trockenkonzentrat (24), wobei der Wechselbehälter (16) einen Ablaufanschluss (17) und einen Kombianschluss (15) aufweist, und
einer stationären Herstellungsanlage (12), die über mindestens zwei Kupplungsanordnungen (30,31) mit dem Ablaufanschluss (17) und dem Kombianschluss (15) fluidisch verbunden ist und durch die das Trockenkonzentrat (24) mit Wasser zu einem flüssigen Dialysekonzentrat gemischt wird,
wobei mindestens eine der Kupplungsanordnungen (30,31) einen anlageseitigen Kupplungskörper (40,50) mit einem im Querschnitt nicht-kreisförmigen Flüssigkeits-Leitungsstecker (42,54) und einem behälterseitigen Kupplungskörper (41,51) mit einer im Querschnitt nicht-kreisförmigen komplementären Flüssigkeits-Leitungsbuchse (43,55) aufweist,
wobei der Flüssigkeits-Leitungsstecker (42,54) und die Flüssigkeits-Leitungsbuchse (43,55) nur in einer einzigen rotatorischen Position ineinander steckbar sind, **dadurch gekennzeichnet dass** die Herstellungsanlage (12) einen Drucksensor (13) aufweist, der fluidisch mit einer Kupplungsanordnung (31) verbunden ist, die anlagenseitig einen Drucksensor- Leitungsstecker (52) und behälterseitig eine korrespondierende Drucksensor- Leitungsbuchse (53) aufweist.

2. Dialysekonzentrat-Herstellungsanordnung (10) nach Anspruch 1, wobei der Flüssigkeits-Leitungsstecker (42,54) und die Flüssigkeits-Leitungsbuchse (43,55) jeweils oval ausgebildet sind.

3. Dialysekonzentrat-Herstellungsanordnung (10) nach Anspruch 2, wobei der Wechselbehälter (16) ein ovales Steigrohr (26) aufweist.

4. Dialysekonzentrat-Herstellungsanordnung (10) nach einem der vorangegangenen Ansprüche, wobei der Flüssigkeits-Leitungsstecker (42,54) außenseitig eine Ringnut (76) aufweist, in der ein flexibler O-Ring (80) sitzt, wobei die Ringnut (76) eine axiale Länge aufweist, die mindestens das 1,5-fache des Durchmessers des Körper des O-Rings (80) beträgt, besonders bevorzugt mindestens das 2,0-fache, so dass der O-Ring (80) beim Einstecken des Flüssigkeits-Leitungssteckers (42,54) in die Flüssigkeits-Leitungsbuchse (43,55) axial in der Ringnut (76) abrollt.

5. Dialysekonzentrat-Herstellungsanordnung (10) nach einem der vorangegangenen Ansprüche, wobei die Kupplungskörper (40,50, 41,51) der Kupplungsanordnung aus Kunststoff bestehen.

6. Dialysekonzentrat-Herstellungsanordnung (10) nach einem der vorangegangenen Ansprüche, wobei der behälterseitige Kupplungskörper (50) einen RFID-Identifikationssender (84) und der anlagenseitige Kupplungskörper (51) ein RFID-Empfänger (82) zum Lesen einer Identifikation aus dem RFID-Identifikationssender (84) aufweist.

7. Wechselbehälter (16) für eine Dialysekonzentrat-Herstellungsanordnung (10) zur Herstellung einer Dialysekonzentrat- Flüssigkeit durch Lösen eines Trockenkonzentrats (24) in Wasser mit den sich auf eine Herstellungsanlage (12) beziehenden Merkmalen der vorangegangenen Ansprüche, wobei der Wechselbehälter (16) die sich auf den Wechselbehälter (16) beziehenden Merkmale der vorangegangenen Ansprüche aufweist.

## Claims

1. Dialysis concentrate production assembly (10) for producing a dialysis concentrate liquid by dissolving a dry concentrate (24) in water, comprising
a mobile interchangeable container (16) with the dry concentrate (24), the interchangeable container (16) having a discharge port (17) and a combined port (15), and
a stationary production system (12) fluidically connected to the discharge port (17) and the combined port (15) via at least two coupling assemblies (30, 31), and by which system the dry concentrate (24) is mixed with water to obtain a liquid dialysis concentrate,
at least one of the coupling assemblies (30, 31) has a system-side coupling body (40, 50) with a liquid line plug (42, 54) of non-circular cross section and a container-side coupling body (41, 51) with a complementary liquid line socket (43, 55) of non-circular cross section,
the liquid line plug (42, 54) and the liquid line socket (43, 55) being configured such that they can be plugged together in only a single rotational position,
**characterized in that**
the production system (12) comprises a pressure sensor (13) fluidically coupled with a coupling assembly (31) which comprises a system-side pressure sensor line connector (52) and a corresponding container-side pressure sensor line socket (53).

2. Dialysis concentrate production assembly (10) of claim 1, wherein the liquid line plug (42, 54) and the liquid line socket (43, 55) are each oval in shape.

3. Dialysis concentrate production assembly (10) of claim 2, wherein the interchangeable container (16) has an oval riser (26).

4. Dialysis concentrate production assembly (10) of one of the preceding claims, wherein the liquid line plug (42, 54) has an annular groove (76) on the outside, in which a flexible O-ring (80) is seated, the annular groove (76) having an axial length that is at least 1.5 times, particularly preferred at least 2.0 times the diameter of the body of the O-ring (80), so that the O-ring (80) rolls axially in the annular groove (76) when the liquid line plug (42, 54) is plugged into the liquid line socket (43, 55).

5. Dialysis concentrate production assembly (10) of one of the preceding claims, wherein the coupling bodies (40, 50, 41, 51) of the coupling assembly are made of plastic material.

6. Dialysis concentrate production assembly (10) of one of the preceding claims, wherein the container-side coupling body (50) has an RFID identification transmitter (84) and the system-side coupling body (51) has a RFID receiver (82) for reading an identification from the RFID identification transmitter (84).

7. Interchangeable container (16) for a dialysis concentrate production assembly (10) for producing a dialysis concentrate liquid by dissolving a dry concentrate (24) in water comprising the features of the preceding claims related to a production system (12), the interchangeable container (16) having the features of the preceding claims related to the interchangeable container (16).

## Revendications

1. Dispositif (10) de production d'un concentré de dialyse destiné à la production d'un liquide de concentré de dialyse par dissolution d'un concentré sec (24) dans de l'eau, comprenant
un conteneur interchangeable (16) avec le concentré sec (24), ledit conteneur interchangeable (16) comprenant un raccord de sortie (17) et un raccord combiné (15), et
un système de production (12) stationnaire en liaison fluidique avec le raccord de sortie (17) et le raccord combiné (15) par au moins deux ensembles de couplage (30, 31) et par lequel le concentré sec (24) et mélangé avec de l'eau pour obtenir un concentre de dialyse liquide,
au moins un des ensembles de couplage (30, 31) ayant un corps de couplage (40, 50), côté système, avec un connecteur de conduit (42, 54) à liquide, dont la section transversale est non-circulaire, et un corps de couplage (41, 51), côté conteneur, avec une douille de conduit (43, 55) à liquide complémentaire, dont la section transversale est non-circulaire,
le connecteur de conduit (42, 54) à liquide et la douille de conduit (43, 55) à liquide ne pouvant être emboîté que dans une seule position rotative,
**caractérisé en ce que**
le système de production (12) comprend un capteur de pression (13) en liaison fluidique avec un ensemble de couplage (31), qui, côté système, comprend un connecteur de ligne (52) du capteur de pression et, côté conteneur, une douille de ligne (53) du capteur de pression correspondante.

2. Dispositif (10) de production d'un concentré de dialyse selon la revendication 1, dans lequel le connecteur de conduit (42, 54) à liquide et la douille de conduit (43, 55) à liquide sont chacun de forme ovale.

3. Dispositif (10) de production d'un concentré de dialyse selon la revendication 2, dans lequel le conteneur interchangeable (16) a un tube montant (26) de forme ovale.

4. Dispositif (10) de production d'un concentré de dialyse selon l'une quelconque des revendications précédentes, dans lequel le connecteur de conduit (42, 54) à liquide a une rainure annulaire (76), côté extérieure, dans laquelle est positionnée un joint annulaire (80) flexible, la rainure annulaire (76) ayant une longueur axiale qui est au moins 1,5 fois, particulièrement préféré au moins 2,0 fois le diamètre du corps du joint annulaire (80), de manière que le joint annulaire (80) roule axialement dans rainure annulaire (76) lorsque le connecteur de conduit (42, 54) est inséré dans la douille de conduit (43, 55) à liquide.

5. Dispositif (10) de production d'un concentré de dialyse selon l'une quelconque des revendications précédentes, dans lequel les corps de couplage (40, 50, 41, 51) dudit ensemble de couplage sont fabriqués de matière plastique.

6. Dispositif (10) de production d'un concentré de dialyse selon l'une quelconque des revendications précédentes, dans lequel le corps de couplage (50), côté système, a un transmetteur d'identification RFID (84) et le corps de couplage (51), côté conteneur, a un récepteur RFID (82) pour lire une identification dans le transmetteur d'identification RFID (84).

7. Conteneur interchangeable (16) pour un dispositif (10) de production d'un concentré de dialyse destiné à la production d'un liquide de concentré de dialyse par dissolution d'un concentré sec (24) dans de l'eau comprenant les caractéristiques des revendications précédentes se référant à un système de production (12), le conteneur interchangeable (16) comportant les caractéristiques des revendications précédentes se référant au conteneur interchangeable (16).
